# EUROPEAN PATENT SPECIFICATION

(11) **EP 3 508 079 B1**
(45) Date of publication and mention of the grant of the patent: **30.12.2020**
(21) Application number: 17845407.0
(22) Date of filing: 29.08.2017
(51) Int. Cl.: A24F 47/00, A24F 40/20, A24F 40/485, A61M 11/00

(54) **PRESS TYPE AIR INJECTION DEVICE**
DRUCKLUFTINJEKTIONSVORRICHTUNG
DISPOSITIF D'INJECTION D'AIR À PRESSION

(30) Priority: 31.08.2016 CN 201610791004
(43) Date of publication of application: 10.07.2019
(73) Proprietor: China Tobacco Yunnan Industrial Co., Ltd, Kunming, Yunnan 650231 (CN)
(72) Inventor: TANG, Jianguo, Kunming Yunnan 650231 (CN); ZHENG, Xudong, Kunming Yunnan 650231 (CN); ZENG, Xu, Kunming Yunnan 650231 (CN); WANG, Ru, Kunming Yunnan 650231 (CN); WANG, Chengya, Kunming Yunnan 650231 (CN); GONG, Weimin, Kunming Yunnan 650231 (CN); LI, Zhiqiang, Kunming Yunnan 650231 (CN); LEI, Ping, Kunming Yunnan 650231 (CN); SHANG, Shanzhai, Kunming Yunnan 650231 (CN); HAN, Jingmei, Kunming Yunnan 650231 (CN); YUAN, Dalin, Kunming Yunnan 650231 (CN); TANG, Shunliang, Kunming Yunnan 650231 (CN); CHEN, Yongkuan, Kunming Yunnan 650231 (CN); LUO, Hongyong, Kunming Yunnan 650231 (CN)
(74) Representative: Gille Hrabal
(86) International application number: PCT/CN2017/099527
(87) International publication number: WO 2018/041105

(56) References cited:
- CN-A- 1 397 381
- CN-A- 1 880 853
- CN-A- 103 859 607
- CN-A- 106 263 038
- CN-A- 106 263 039
- CN-U- 206 025 227
- CN-Y- 2 211 171
- CN-Y- 201 067 713
- GB-A- 2 285 396

## Description

### Technical Field

The present invention belongs to the field of novel cigarettes and electronic cigarettes, and in particular, relates to a press and snorting type smoking paraphernalia for heating cigarettes, and further to a press puffing device.

### Background

The drip tip of the existing smoking paraphernalia for novel cigarette is generally made of plastic or metal, and a layer of chemical substance is electroplated or sprayed on the surface of the plastic or metal material to form a solidified protective layer (such as a metal plating layer, a paint coating layer). The smoking paraphernalia may be subjected to a pyrolysis at a certain temperature to generate harmful substances that affect human health. In some occasions, the smoke generated by the smoking paraphernalia is banned; the common smoking paraphernalia for novel cigarette is designed with airflow channels from the air inlet to the heating chamber and then to the air outlet. The conductive heating method is used in the heating chamber of the common smoking paraphernalia for novel cigarette, namely, the heating element is in direct contact with the novel cigarette, and the heat generated by the heating element is directly transmitted to the novel cigarette.

One of the disadvantages of the prior art lies in the smoking mode. People smoke through the mouth by directly contacting the cigarette filter, and the material on the surface of the cigarette filter may be subjected to a pyrolysis at a certain temperature to produce harmful substances that will get inhaled. Therefore, it is desirable avoid the direct contact with the cigarette filter while smoking.

Another disadvantage of the prior art lies in the heating mode. The conductive heating method is used in the heating chamber of the common smoking paraphernalia for the novel cigarette, namely, a heating element (such as a metal heating wire) and a high temperature resistant material (such as ceramic) are sintered at a high temperature to form a cigarette heating tube, which transfers the heat directly to the surface of the cigarette. When the tightness between the inner wall of the heating chamber and the cigarette is not uniform, it is prone to generate over heating at the position where the inner wall of the heating chamber and the cigarette is too tight, resulting in burnt smell. Besides, another heating mode of the ordinary cigarette is designed to integrate the printing heating material and Flexible Printed Circuit (FPC) board on the outer wall of the cigarette heating tube, which makes it easy to dissipate the heat generated by the heating material through the medium material and the thermal insulation material, thereby causing heat loss.

The present invention is aimed at solving the above-mentioned problems. CN 2 211 171 Y discloses a spraying cigarette case for giving-up smoking. A piston body is arranged on the upper end of a casing. The piston body is connected with the casing and seals the port of the casing. An air cylinder is arranged on one side of the piston body which is arranged on the port and is communicated with the inner cavity of the casing; a piston is arranged in the air cylinder and is connected with the pressing plug which is arranged on the upper part of the piston by connecting-rods. A spring is arranged between the top of the air cylinder and the pressing plug; the lower end of a cover is sheathed on the piston body. A suction pipe is arranged on the other side of the port of the casing and is communicated with an inner cavity.

### Summary

In view of the disadvantages of the existing cigarette smoking paraphernalia, a press and snorting type smoking paraphernalia for heating a cigarette is designed in the present invention, namely, the smoke from the smoke outlet of the press and snorting type smoking paraphernalia for the novel cigarette is inhaled by the nose, rather than the mouth directly contacting the cigarette filter, so as to achieve the non-contact smoking. The present diclosure also describes: A. a roasting-type heating device, including:
a cigarette heating tube 15, wherein a plurality of wall holes 151 are provided on a side wall of the cigarette heating tube; a heating element 6, wherein the heating element is disposed on an outer side of the cigarette heating tube 15 and is not in contact with the cigarette heating tube; and a thermal insulation pipe 7, wherein the thermal insulation pipe is sleeved on the outer side of the heating element 6 and is not in contact with the heating element.

A first aspect of the present invention relates to a press and snorting type smoking paraphernalia for heating the cigarette, including:
B. a press puffing device, including:
a piston support 2 including a left cylindrical cavity 21, a right cylindrical cavity 22, and a connection portion 23 between the left cylindrical cavity and the right cylindrical cavity, wherein both upper and lower ends of the left cylindrical cavity 21 and the right cylindrical cavity 22 are open;
a nozzle holder 12, including a cylindrical inner cavity 123 with a roof, wherein a bottom opening of the inner cavity 123 is connected to the cigarette heating tube 15 for air to pass through; a lateral gas channel 124 is provided on a side wall of the inner cavity 123; the lateral gas channel 124 is connected to an air outlet 121 for air to pass through; and a nozzle holder guide slot 122 penetrating the side wall of the cylindrical inner cavity 123 is provided thereon; and
a sealing fixer 14 including a left cavity 141, a right cavity 142 and a partition wall 143 between the left cavity and the right cavity, wherein a sealing fixer sliding slot 144 is provided on the partition wall 143; and an inner shape of the left cavity 141 is matched with an outer shape of the nozzle holder 12;
wherein, positional relationships of various components in the press puffing device are as follows:
   the nozzle holder 12 is contained in the left cavity 141 of the sealing fixer 14; the right cylindrical cavity 22 of the piston support 2 is contained in the right cavity 142; the sealing fixer sliding slot 144 and the nozzle holder guide slot 122 are configured to overlap each other; the connection portion 23 of the piston support 2 is allowed to pass through and slide upwards and downwards in the sealing fixer sliding slot 144 and the nozzle holder guide slot 122; the left cylindrical cavity 21 of the piston support 2 is contained in the cylindrical inner cavity 123 of the nozzle holder 12; a top end of the left cylindrical cavity 21 can be appressed or not appressed with the roof of the cylindrical inner cavity 123; a side wall of the left cylindrical cavity 21 closes the lateral gas channel 124 when the top end of the left cylindrical cavity 21 is appressed with the roof, and opens the lateral gas channel 124 when the top end of the left cylindrical cavity 21 is not appressed with the roof.

Preferably, a sealing ring 13 is sleeved between the left cylindrical cavity 21 of the piston support 2 and the cylindrical inner cavity 123 of the nozzle holder 12.

Preferably, a spring 3 is contained in the right cavity 142 of the sealing fixer 14; one end of the spring abuts against a lower end wall of the right cylindrical cavity 22 of the piston support 2, and other end of the spring abuts against a bottom wall of the sealing fixer 14; when a user no longer presses the piston support 2, the piston support 2 is bounced back by a reset action of the spring.

Preferably, a radial inner edge 24 is provided at a top end opening of the left cylindrical cavity 21 of the piston support 2. Such arrangement of the inner edge 24 is aimed at increasing a compression area of gas in the cylindrical inner cavity 123 when the left cylindrical cavity 21 moves downward, thereby improving an effect of compressing the smoke.

Preferably, a power supply start button 1 and a switch control board 4 are contained in the right cylindrical cavity 22 of the piston support 2, and are configured to be contactable mutually by contact points; the power supply start button 1 is configured to control the energization of the heating element 6.

Preferably, the heating element 6 is spiral.

A second aspect of the present invention relates to a press puffing device including the following components:
a piston support 2 including a left cylindrical cavity 21, a right cylindrical cavity 22, and a connection portion 23 between the left cylindrical cavity and the right cylindrical cavity, wherein both upper and lower ends of the left cylindrical cavity 21 and the right cylindrical cavity 22 are open;
a nozzle holder 12, including a cylindrical inner cavity 123 with a roof, wherein a lateral gas channel 124 is provided on a side wall of the inner cavity 123; the lateral gas channel 124 is connected to an air outlet 121 for air to pass through; and a nozzle holder guide slot 122 penetrating the side wall of the cylindrical inner cavity 123 is provided thereon; and
a sealing fixer 14 including a left cavity 141, a right cavity 142 and a partition wall 143 between the left cavity and the right cavity, wherein a sealing fixer sliding slot 144 is provided on the partition wall 143; and an inner shape of the left cavity 141 is matched with an outer shape of the nozzle holder 12;
wherein, positional relationships of various components are as follows:
   the nozzle holder 12 is contained in the left cavity 141 of the sealing fixer 14; the right cylindrical cavity 22 of the piston support 2 is contained in the right cavity 142; the sealing fixer sliding slot 144 and the nozzle holder guide slot 122 are configured to overlap each other; the connection portion 23 of the piston support 2 is allowed to pass through and slide upwards and downwards in the sealing fixer sliding slot 144 and the nozzle holder guide slot 122; the left cylindrical cavity 21 of the piston support 2 is contained in the cylindrical inner cavity 123 of the nozzle holder 12; a top end of the left cylindrical cavity 21 can be appressed or not appressed with the roof of the cylindrical inner cavity 123; the lateral gas channel 124 is closed when the top end of the left cylindrical cavity 21 is appressed with the roof, and the lateral gas channel 124 is opened when top end of the left cylindrical cavity 21 is not appressed with the roof.

### Advantages of the present invention:

1. The complicated airflow channel design of the smoking paraphernalia for traditional electrically heated cigarette is omitted, which reduces the processing cost of the equipment. In the smoking paraphernalia for traditional electrically heated cigarette, the air enters the cigarette heating tube along the airflow channel in the smoking paraphernalia and takes out the smoke generated by heating the cigarette, so that the user can smoke. However, the complete design of airflow channel is required to design air inlet holes connected to each other for air to pass through between a plurality of components adjacent to each other, which increases the difficulty of manufacturing components. In the present patent, the cigarette heating tube is ventilated in a one-way direction, and the air fails to enter the heating chamber from the bottom or the side of the heating chamber, the smoke formed in the heating chamber is spurted under the pressure of the piston support due to the increase in pressure, which omits the airflow channel design and decreases the manufacturing difficulty.
2. The heating element works under a radiant heating mode instead of directly contacting the cigarette, leading to uniform heating, and avoiding local overheating.
3. Since the smoke is spurted out under the compression action from the smoking paraphernalia for snorting by the user, without the necessity of using mouth, the cigarette filter is not required, thereby naturally avoiding the contact between the mouth and the cigarette filter, which is convenient and hygienic, and can reduce the direct damage of nicotine in the smoke to the human oral mucosa.

### Brief Description of the Drawings

Fig. 1 is a structural schematic diagram showing a press and snorting type smoking paraphernalia for heating a cigarette of the present invention.
Fig. 2 is an exploded view showing various components of a press and snorting type smoking paraphernalia for heating a cigarette of the present invention.
Fig. 3 is a structural schematic diagram showing a roasting-type heating device (namely, portion D in Fig. 1) of a press and snorting type smoking paraphernalia for heating a cigarette of the present invention.
Fig. 4 is an exploded view showing a roasting-type heating device of a press and snorting type smoking paraphernalia for heating a cigarette of the present invention.
Fig. 5 is a cross-sectional view showing a structure of a press and snorting type smoking paraphernalia for heating a cigarette of the present invention in a non-working state.
Fig. 6 is an enlarged view showing a portion indicated by a circle A in Fig. 5.
Fig. 7 is a cross-sectional view showing a structure of a press and snorting type smoking paraphernalia for heating a cigarette of the present invention when the heating start button 1 is pressed and the piston support 2 is not pressed.
Fig. 8 is an enlarged view showing a portion indicated by a circle B in Fig. 7.
Fig. 9 is a cross-sectional view showing a structure of a press and snorting type smoking paraphernalia for heating a cigarette of the present invention when the piston support 2 is pressed.
Fig. 10 is an enlarged view showing a portion indicated by a circle C in Fig. 9.

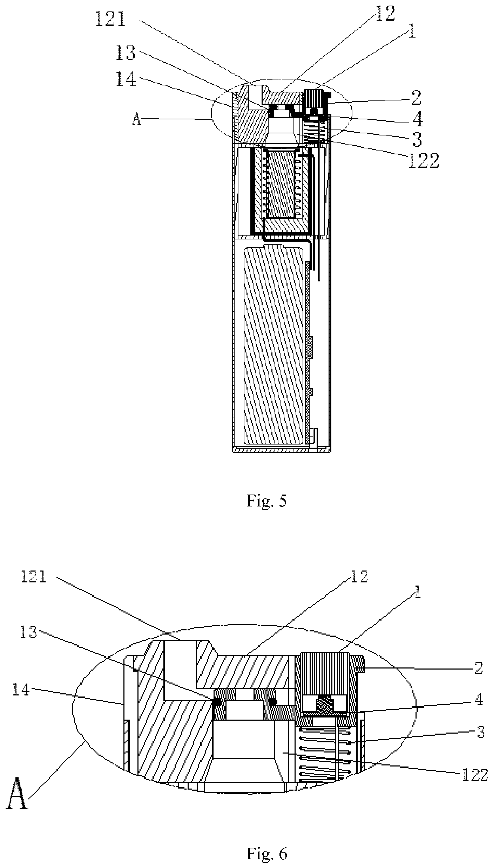

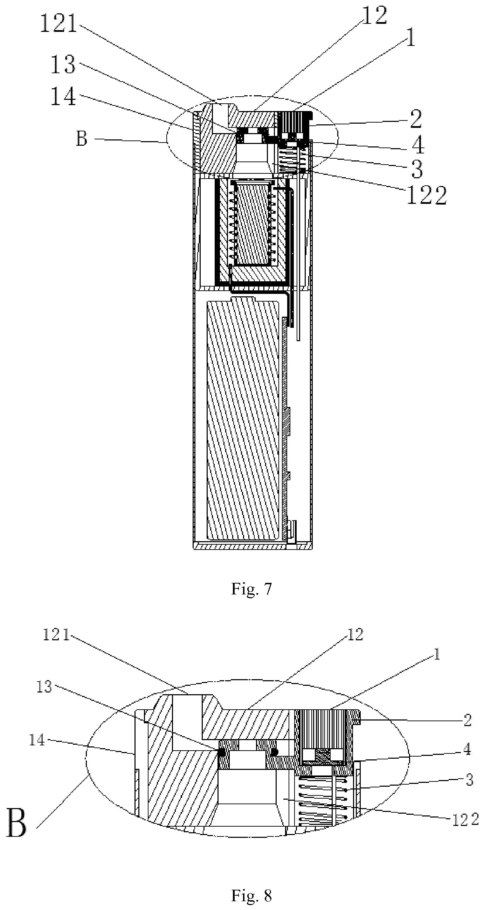

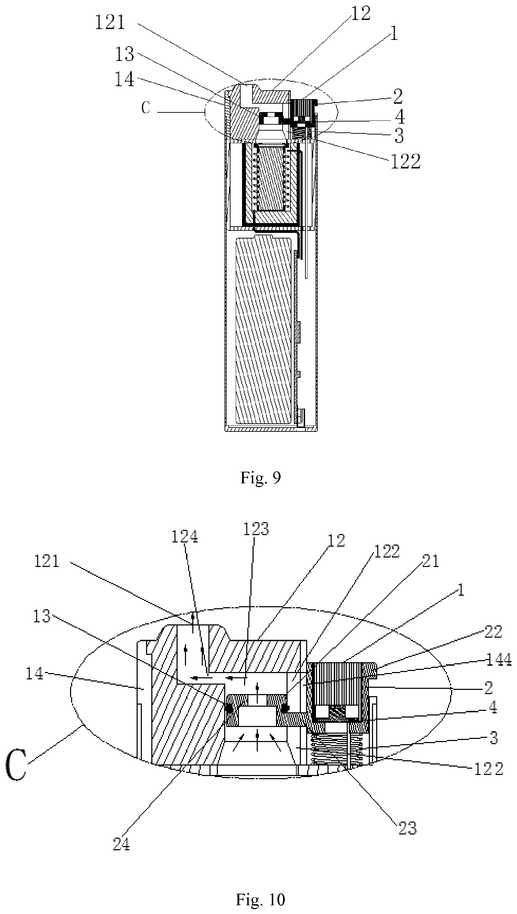

Wherein Figs. 6, 8 and 10 are also structural schematic diagrams of a press puffing device of the present invention.

The reference signs are illustrated as follows:
1: heating start button; 2: piston support; 3: spring; 4: switch control board; 5: cigarette; 6: heating element; 7: thermal insulation pipe; 8: main control circuit board; 9: USB charging port; 10: battery; 11: base; 12: nozzle holder; 13: sealing ring; 14: sealing fixer; 15: cigarette heating tube; 16: roasting-type heating device housing; 17: porous thermal insulation material; 18: housing;
21: left cylindrical cavity; 22: right cylindrical cavity; 23: connection portion; 24: radial inner edge;
121: air outlet; 122: nozzle holder guide slot; 123: cylindrical inner cavity; 124: lateral gas channel; 141: left cavity; 142: right cavity; 143: partition wall; 144: sealing fixer sliding slot; 151: wall hole.

### Detailed Description of the Embodiments

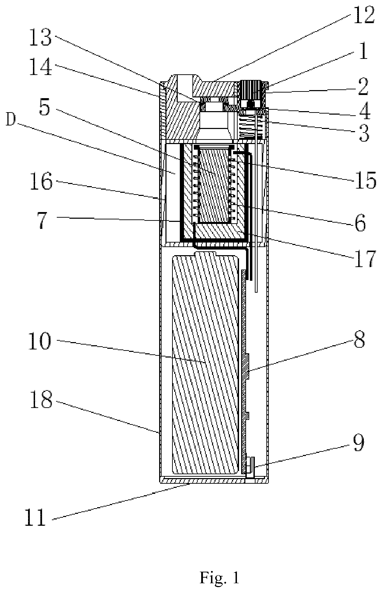

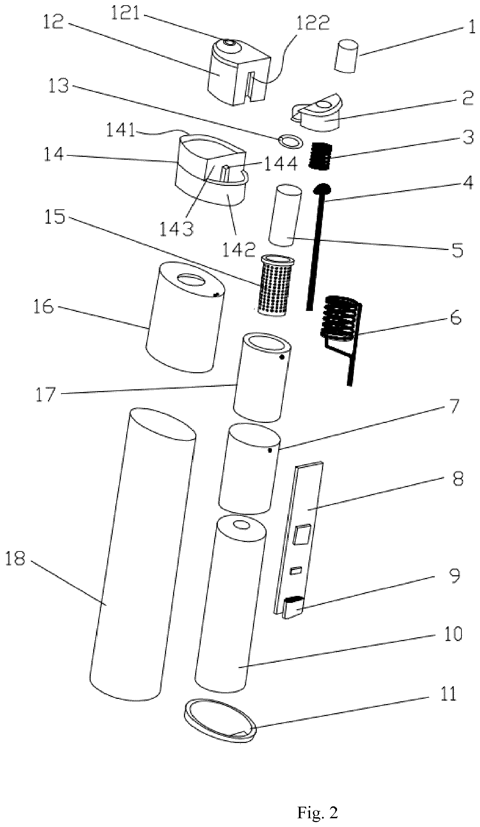

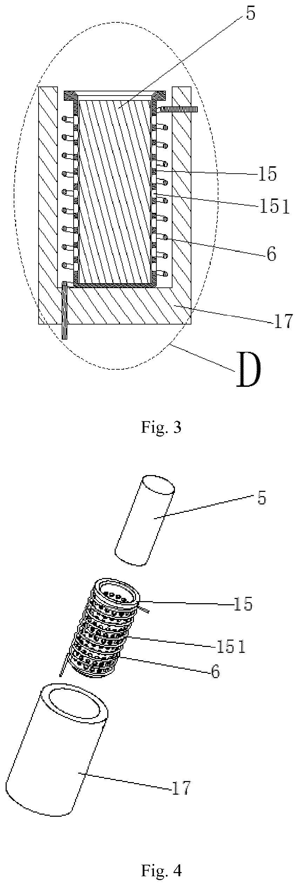

The overall structure of the press and snorting type smoking paraphernalia for heating the cigarette of the present invention is described in the Summary, and the related components and structures thereof can be clearly shown in Figs. 1-4, which will not be described repeatedly hereinafter.

The working process of the press and snorting type smoking paraphernalia for heating the cigarette of the present invention will be described hereinafter with reference to the drawings:
A solid tobacco material which can be heated by roasting and subjected to a pyrolysis to produce an aroma material is used in the smoking paraphernalia. The solid tobacco material is specially formulated and processed into a cylindrical cigarette. The cigarette 5 is placed in the cigarette heating tube 15.

When the user presses the heating start button 1 and does not press the piston support 2, the heating element 6 suspended between the cigarette heating chamber 15 and the thermal insulation pipe 7 is energized to start generating heat. The heat generated by the heating element is transmitted to the surface of the cigarette 5 through the wall hole 151 on the cigarette heating tube 15 in the radiated manner, and gradually penetrates into the center of the cigarette 5. Meanwhile, the cigarette heating tube also receives the radiation heat from the heating element to be heated, and conductively heats the cigarette contained in the cigarette heating tube. The tobacco material volatilizes the aroma gas after being heated. The aroma gas is contained in the cylindrical inner cavity 123.

Since the user has not pressed the piston support 2 at this time, the top end of the left cylindrical cavity 21 is appressed with the roof of the cylindrical inner cavity 123, and the side wall of the left cylindrical cavity 21 closes the lateral gas channel 124. In this way, the cylindrical inner cavity 123 is in a sealed state, so that the aroma gas remains inside the cylindrical inner cavity.

When the user presses the piston support 2 downwards, the left cylindrical cavity 21 moves downwards accordingly, so that the top end of the left cylindrical cavity is not appressed with the roof of the cylindrical inner cavity 123. In this way, the side wall of the left cylindrical cavity 21 does not block the lateral gas channel 124, thereby the lateral gas channel 124 is opened. Meanwhile, the volume of the cylindrical inner cavity 123 is reduced, so that the smoke contained in the cylindrical inner cavity is compressed and flows to the air outlet 121 through the lateral gas channel 124, and then is spurted out for snorting by the user. The spring 3 is also compressed while the user presses the piston support 2 downwards.

When the user releases the piston support 2, the piston support 2 returns to the original position under the resilience of the spring 3.

When the user wants to smoke next time, repeat the above-mentioned processes.

## Claims

1. A press puffing device comprising the following components:
a piston support (2), comprising a left cylindrical cavity (21), a right cylindrical cavity (22), and a connection portion (23) between the left cylindrical cavity and the right cylindrical cavity, wherein both upper and lower ends of the left cylindrical cavity (21) and the right cylindrical cavity (22) are open;
a nozzle holder (12), comprising a cylindrical inner cavity (123) with a roof, wherein a lateral gas channel (124) is provided on a side wall of the cylindrical inner cavity (123); the lateral gas channel (124) is connected to an air outlet (121) for air to pass through; and a nozzle holder guide slot (122) penetrating the side wall of the cylindrical inner cavity (123) is provided thereon; and
a sealing fixer (14), comprising a left cavity (141), a right cavity (142) and a partition wall (143) between the left cavity and the right cavity, wherein a sealing fixer sliding slot (144) is provided on the partition wall (143); and an inner shape of the left cavity (141) is matched with an outer shape of the nozzle holder (12);
wherein, positional relationships of various components are as follows:
the nozzle holder (12) is contained in the left cavity (141) of the sealing fixer (14); the right cylindrical cavity (22) of the piston support (2) is contained in the right cavity (142); the sealing fixer sliding slot (144) and the nozzle holder guide slot (122) are configured to overlap each other; the connection portion (23) of the piston support (2) is allowed to pass through and slide upwards and downwards in the sealing fixer sliding slot (144) and the nozzle holder guide slot (122); the left cylindrical cavity (21) of the piston support (2) is contained in the cylindrical inner cavity (123) of the nozzle holder (12); a top end of the left cylindrical cavity (21) can be appressed or not appressed with the roof of the cylindrical inner cavity (123); the lateral gas channel (124) is closed when the top end of the left cylindrical cavity (21) is appressed with the roof, and the lateral gas channel (124) is opened when the top end of the left cylindrical cavity (21) is not appressed with the roof.

2. The press puffing device according to claim 1, **characterized in that** a sealing ring (13) is sleeved between the left cylindrical cavity (21) of the piston support (2) and the cylindrical inner cavity (123) of the nozzle holder (12).

3. The press puffing device according to claim 1, **characterized in that** a spring (3) is contained in the right cavity (142) of the sealing fixer (14); one end of the spring abuts against a lower end wall of the right cylindrical cavity (22) of the piston support (2); and other end of the spring abuts against a bottom wall of the sealing fixer (14).

4. The press puffing device according to claim 1, **characterized in that** a radial inner edge (24) is provided in a top opening of the left cylindrical cavity (21) of the piston support (2).

## Patentansprüche

1. Ein Pressdampfgerät umfassend die folgenden Komponenten:
einen Kolbenträger (2), der einen linken zylindrischen Hohlraum (21), einen rechten zylindrischen Hohlraum (22) und einen Verbindungsabschnitt (23) zwischen dem linken zylindrischen Hohlraum und dem rechten zylindrischen Hohlraum umfasst, wobei sowohl das obere als auch das untere Ende des linken zylindrischen Hohlraums (21) und des rechten zylindrischen Hohlraums (22) offen sind;
einen Düsenhalter (12), der einen zylindrischen Innenhohlraum (123) mit einem Dach umfasst, wobei ein seitlicher Gaskanal (124) an einer Seitenwand des zylindrischen Innenhohlraums (123) vorgesehen ist; der seitliche Gaskanal (124) mit einem Luftauslass (121) verbunden ist, durch den Luft strömen kann; und ein Düsenhalter-Führungsschlitz (122), der die Seitenwand des zylindrischen Innenhohlraums (123) durchdringt, daran vorgesehen ist; und
einen Dichtungsfixierer (14), der einen linken Hohlraum (141), einen rechten Hohlraum (142) und eine Trennwand (143) zwischen dem linken Hohlraum und dem rechten Hohlraum umfasst, wobei ein Dichtungsfixierergleitschlitz (144) an der Trennwand (143) vorgesehen ist; und eine innere Form des linken Hohlraums (141) an eine äußere Form des Düsenhalters (12) angepasst ist;
wobei Positionsbeziehungen verschiedener Komponenten wie folgt sind:
der Düsenhalter (12) ist in dem linken Hohlraum (141) des Dichtungsfixierers (14) enthalten; der rechte zylindrische Hohlraum (22) des Kolbenträgers (2) ist in dem rechten Hohlraum (142) enthalten; der Dichtungsfixierergleitschlitz (144) und der Düsenhalter-Führungsschlitz (122) sind dazu eingerichtet, einander zu überlappen; der Verbindungsabschnitt (23) des Kolbenträgers (2) kann durch den Dichtungsfixierergleitschlitz (144) und den Düsenhalter-Führungsschlitz (122) hindurchgehen und darin aufwärts und abwärts gleiten; der linke zylindrische Hohlraum (21) des Kolbenträgers (2) ist in dem zylindrischen Innenhohlraum (123) des Düsenhalters (12) enthalten; ein oberes Ende des linken zylindrischen Hohlraums (21) kann mit dem Dach des zylindrischen Innenhohlraums (123) unter Druck gesetzt oder nicht unter Druck gesetzt werden; der seitliche Gaskanal (124) ist geschlossen, wenn das obere Ende des linken zylindrischen Hohlraums (21) mit dem Dach unter Druck gesetzt ist, und der seitliche Gaskanal (124) ist geöffnet, wenn das obere Ende des linken zylindrischen Hohlraums (21) nicht mit dem Dach unter Druck gesetzt ist.

2. Das Pressdampfgerät gemäß Anspruch 1, **dadurch gekennzeichnet, dass** ein Dichtungsring (13) zwischen dem linken zylindrischen Hohlraum (21) des Kolbenträgers (2) und dem zylindrischen Innenhohlraum (123) des Düsenhalters (12) ummantelt ist.

3. Das Pressdampfgerät gemäß Anspruch 1, **dadurch gekennzeichnet, dass** eine Feder (3) in dem rechten Hohlraum (142) des Dichtungsfixierers (14) enthalten ist; ein Ende der Feder an einer unteren Endwand des rechten zylindrischen Hohlraums (22) des Kolbenträgers (2) anliegt; und das andere Ende der Feder an einer Bodenwand des Dichtungsfixierers (14) anliegt.

4. Das Pressdampfgerät gemäß Anspruch 1, **dadurch gekennzeichnet, dass** eine radiale Innenkante (24) in einer oberen Öffnung des linken zylindrischen Hohlraums (21) des Kolbenträgers (2) vorgesehen ist.

## Revendications

1. Dispositif de soufflage à pression comprenant les composants suivants :
un support de piston (2) comprenant une cavité cylindrique gauche (21), une cavité cylindrique droite (22), et une partie de liaison (23) entre la cavité cylindrique gauche et la cavité cylindrique droite, dans lequel les extrémités supérieures et les extrémités inférieures de la cavité cylindrique gauche (21) et de la cavité cylindrique droite (22) sont ouvertes ;
un support de buse (12) comprenant une cavité intérieure cylindrique (123) avec un toit, dans lequel un canal de gaz latéral (124) est disposé sur une paroi latérale de la cavité intérieure cylindrique (123) ; le canal de gaz latéral (124) est relié à une sortie d'air (121) pour que l'air puisse la traverser ; et une fente de guidage de soutien de buse (122), qui pénètre la paroi latérale de la cavité intérieure cylindrique (123), est disposée sur celle-ci ; et
un fixateur d'étanchéité (14) comprenant une cavité gauche (141), une cavité droite (142) et une cloison de séparation (143) entre la cavité gauche et la cavité droite, dans lequel une fente de glissement de fixateur d'étanchéité (144) est disposée sur la cloison de séparation (143) et une forme intérieure de la cavité gauche (141) correspond à la forme extérieure du support de buse (12) ;
dans lequel des relations de position de divers composants sont les suivantes :
le support de buse (12) est contenu dans la cavité gauche (141) du fixateur d'étanchéité (14) ; la cavité cylindrique droite (22) du support de piston (2) est contenu dans la cavité droite (142) ; la fente de glissement de fixateur d'étanchéité (144) et la fente de guidage de soutien de buse (122) sont configurées pour se chevaucher l'une par rapport à l'autre; la partie de liaison (23) du support de piston (2) peut passer à travers et glisser vers le haut et vers le bas dans la fente de glissement de fixateur d'étanchéité (144) et la fente de guidage de soutien de buse (122) ; la cavité cylindrique gauche (21) du support de piston (2) est contenue dans la cavité cylindrique intérieure (123) du support de buse (12) ; une extrémité supérieure de la cavité cylindrique gauche (21) peut être pressée ou ne peut pas être pressée contre le toit de la cavité cylindrique intérieure (123) ; le canal de gaz latéral (124) est fermé, si l'extrémité supérieure de la cavité cylindrique gauche (21) est pressée contre le toit et le canal de gaz latéral (124) est ouvert, si l'extrémité supérieure de la cavité cylindrique gauche (21) n'est pas pressée contre le toit.

2. Dispositif de soufflage à pression selon la revendication 1, **caractérisé en ce qu'**une bague d'étanchéité (13) est disposée entre la cavité cylindrique gauche (21) du support de piston (2) et la cavité cylindrique intérieure (123) du support de buse (12).

3. Dispositif de soufflage à pression selon la revendication 1, **caractérisé en ce qu'**un ressort (3) est contenu dans la cavité droite (142) du fixateur d'étanchéité (14) ; une extrémité du ressort s'appuie contre une paroi d'extrémité inférieure de la cavité cylindrique droite (22) du support de piston (2) ; et l'autre extrémité du ressort s'appuie contre une paroi de fond du fixateur d'étanchéité (14).

4. Dispositif de soufflage à pression selon la revendication 1, **caractérisé en ce qu'**un bord radial intérieur (24) est disposé dans une ouverture supérieure de la cavité cylindrique gauche (21) du support de piston (2).
